# EUROPÄISCHE PATENTSCHRIFT

(11) **EP 2 823 855 B1**
(45) Veröffentlichungstag und Bekanntmachung des Hinweises auf die Patenterteilung: **23.03.2016**
(21) Anmeldenummer: 14002156.9
(22) Anmeldetag: 24.06.2014
(51) Int. Cl.: A61N 1/36

(54) **Stimulationsvorrichtung**
Stimulation device
Dispositif de stimulation

(30) Priorität: 10.07.2013 DE 102013011541
(43) Veröffentlichungstag der Anmeldung: 14.01.2015
(73) Patentinhaber: cerbomed GmbH, 91052 Erlangen (DE)
(72) Erfinder: Frenkel, Wolf Gerhard, 72514 Inzigkofen-Engelswies (DE); Zschaeck, Thomas, 90427 Nürnberg (DE); Hartlep, Andreas, 83607 Holzkirchen (DE)
(74) Vertreter: Gosdin, Michael

(56) Entgegenhaltungen:
- DE-A1-102011 018 228
- DE-B3-102010 054 165
- DE-B4-102005 003 735
- US-A- 5 458 625
- US-A- 5 514 175
- US-A- 6 119 044
- US-A1- 2008 249 594

## Beschreibung

Die Erfindung betrifft eine Stimulationsvorrichtung zur Aufbringung eines transkutanen elektrischen Stimulationsreizes auf die Oberfläche eines Abschnittes des menschlichen Ohrs, die ein am oder im Ohr anbringbares Halteelement sowie mindestens zwei Elektroden aufweist, die in oder an einem Elektrodenträger angeordnet sind.

Eine gattungsgemäße Stimulationsvorrichtung ist aus der US 5 514 175 A bekannt. Die US 2008/0021517 A1 zeigt eine ähnliche Lösung.

Es ist generell bekannt, durch invasive und non-invasive Reizung der Nerven Einfluss auf deren neurophysiologische und neuroelektrische Qualität und damit auf die Funktion der stimulierten Nerven zu nehmen. Hierdurch können verschiedene Krankheitszustände behandelt werden. Es existieren zahlreiche Vorrichtungen sowohl zur invasiven als auch zur non-invasiven Stimulation.

Die vorliegende Erfindung stellt auf die Methode der transkutanen elektrischen Nervenstimulation ab. Bei diesem Verfahren werden Impulsströme verschiedener Stromformen, Amplituden, Impulsdauern und Frequenzen durch die Haut hindurch an verschiedenen Nerven appliziert und verändern deren Statusparameter in vorteilhafter Weise.

Eine Stimulationsvorrichtung der eingangs genannten Art ist aus der DE 10 2010 054 165 B3 bekannt. Hier ist eine Elektrodenanordnung beschrieben, die ein am Ohr anbringbares Halteelement aufweist. An diesem ist über einen elastischen Abschnitt ein Elektrodenträger angeordnet, der zwei Elektroden hält. Der Elektrodenträger besteht aus elektrisch nicht-leitendem Kunststoff und trägt die beiden Elektroden aus Metall.

Eine andere Bauform einer Stimulationsvorrichtung ist aus der EP 2 026 872 B1 bekannt. Hier ist eine Stimulationsvorrichtung beschrieben, die ein vollständig in der Pinna (Ohrmuschel) unterbringbares Gehäuse umfasst. Von diesem aus erstrecken sich zwei gebogene, drahtförmige Abschnitte, wobei diese als federelastische Halterungen ausgebildet sind. Damit kann die Elektrodenanordnung durch sanftes Einklemmen in der Pinna in die benötigte Position gebracht werden, so dass der Gehörgang mit einem transkutanen Stimulationsreiz beaufschlagt werden kann.

Wenngleich die vorbekannten Stimulationsvorrichtungen bereits zu guten Behandlungsergebnissen führen, haben sich in der Praxis gewisse Nachteile der Anordnung herausgestellt. Hierbei ist die Kontaktqualität der Elektroden zu nennen, die mitunter noch nicht optimal ist, da die von den Elektroden akquirierten Hautareale nicht ausreichend groß sind. Die Erhöhung des Anpressdrucks auf die Hautoberfläche ist zumeist keine geeignete Maßnahme zur Behebung dieser Schwierigkeiten, da hierdurch die Haut des Patienten zu stark belastet wird.

Der Erfindung liegt daher die Aufgabe zugrunde, eine Stimulationsvorrichtung der gattungsgemäßen Art so fortzubilden, dass die genannten Nachteile überwunden werden. Es soll also eine Weiterbildung dahingehend vorgeschlagen werden, dass die Kontaktqualität der Elektroden verbessert wird, ohne die Hautoberfläche des Patienten stark zu belasten, so dass ein hoher Tragekomfort erreichbar ist.

Die Lösung dieser Aufgabe durch die Erfindung ist dadurch gekennzeichnet, dass das Halteelement für den Elektrodenträger aus zwei Teilen besteht, wobei das eine Teil als Haltebügel ausgebildet ist, der zum Platzieren zwischen der Oberfläche des Kopfs und der Pinna des Trägers der Stimulationsvorrichtung ausgebildet ist, wobei das andere Teil als Verbindungselement zwischen dem Haltebügel und dem Elektrodenträger ausgebildet ist, wobei zwischen dem Haltebügel und dem Verbindungselement ein Scharniergelenk angeordnet ist, das bei bestimmungsgemäßer Benutzung der Stimulationsvorrichtung eine Verschwenkung des Verbindungselements relativ zum Haltebügel um eine Achse erlaubt, die parallel oder unter einem Winkel von weniger als 30° zur Geradeausblickrichtung des Trägers (sagittale Achse) der Stimulationsvorrichtung ist.

Insofern sieht die Erfindung eine Stimulationsvorrichtung mit einer zweiteiligen Halterung vor, wobei das eine Teil hinter dem Ohr eingehängt werden kann und das andere Teil den Elektrodenträger mit den Elektroden hält. Die beiden besagten Teile sind scharnierartig, d. h. relativ zueinander schwenkbar beweglich, wobei die Achse der Schwenkbewegung im wesentlichen der Geradeausblickrichtung bzw. Sagittalrichtung entspricht (man könnte also auch sagen, dass diese Richtung bei gerade gehaltenem Kopf senkrecht auf dem Brustkorb stehend horizontal nach vorne gewandt ist). Der Elektrodenträger mit den Elektroden bewegt sich bei dieser Verschwenkung demgemäß im wesentlichen in transversale Richtung, d. h. senkrecht auf das Schläfenbein zu.

Zu den hier relevanten Richtungen sei noch angemerkt, dass die Geradeausblickrichtung in der anatomischen Nomenklatur auch als sagittale Richtung bezeichnet wird. Die horizontale Richtung senkrecht hierzu ist die transversale Richtung; die Richtung, die sowohl senkrecht auf der sagittalen Richtung als auch auf der transversalen Richtung steht, ist die longitudinale Richtung (die also beim aufrecht stehenden Menschen vom Kopf zu den Füßen weist).

Das Scharniergelenk kann dabei mittels eines Federelements in eine Verschwenkrichtung vorgespannt sein. Diese Vorspannung kann öffnend oder schließend vorgesehen werden, d. h. sie kann das Verbindungselement in die bestimmungsgemäße Benutzungsposition hinein bewegen oder aus dieser heraus. Bei bestimmungsgemäßem Gebrauch wird die Wirkung dieses Federelements genutzt, um den Andruck des am Haltebügel scharnierartig angeordneten Verbindungselements samt Elektrodenträger so einzustellen, dass der Elektrodenträger und damit auch die Elektroden mit leichtem Druck auf die Hautoberfläche des zu stimulierenden Hautareals gepresst werden.

Bevorzugt sind hierbei Mittel vorhanden, mit denen die Vorspannung des Federelements eingestellt werden kann.

Der Elektrodenträger ist bevorzugt starr, d. h. ohne Möglichkeit der Ausübung einer Relativbewegung, am einen Ende des Verbindungselements befestigt. Allerdings ist die Befestigung vorzugsweise als lösbare Befestigung ausgebildet, so dass der Elektrodenträger abgenommen werden kann. Die Befestigung des Elektrodenträgers am Verbindungselement kann hierbei bevorzugt über eine formschlüssige Verbindung, insbesondere über eine Schwalbenschwanznut, erfolgen, die in den Elektrodenträger oder in das Verbindungselement eingearbeitet ist und die mit einem kongruenten Gegenstück zusammenwirkt, das am Verbindungselement oder am Elektrodenträger angeformt ist.

Der Haltebügel kann sichelförmig ausgebildet sein und an seinem einen Ende das Scharniergelenk aufweisen.

Der Haltebügel hat gemäß einer bevorzugten Ausführungsform der Erfindung eine Seele aus plastisch verformbarem Material, insbesondere aus Metall. Diese kann von einem Ummantelungsmaterial, insbesondere von einem biokompatiblen Kunststoffmaterial, besonders bevorzugt von Silikon, von Polyamid, von Polypropylen oder von Polyurethan, umgeben sein.

Das Ummantelungsmaterial kann auch aus einem geschlossenporigen Schaum bestehen, der sich in den Spalt zwischen Ohr und Schädel schmiegt. Das Ummantelungsmaterial kann an seiner Oberfläche zusätzlich eine erhöhte Haftfähigkeit bis hin zur Klebung besitzen. Dadurch findet es zusätzlichen Halt, um als Widerlager für die Vorspannung des Elektrodenträgers gegen die Haut der Cymba zu dienen. Das Ummantelungsmaterial kann in einer bevorzugten Ausführungsart auswechselbar sein. Die Seele des Haltebügels kann auch aus einem Metall mit Formgedächtnis (Memoryfunktion; beispielsweise Nitinol) bestehen, das z. B. ab einer Temperatur von 28 °C seine Biegung und damit die Vorspannung bzw. den Andruck des Elektrodenträgers gegen die Haut verstärkt. In einer weiteren Ausführung kann der Haltebügel seinerseits mit Elektroden ausgestattet sein, beispielsweise mit Gegenelektroden zu den Elektroden des bereits genannten Elektrodenträgers. In diesem Fall ergibt sich eine transmurale Durchströmung des Gewebes mit entsprechend intensiverer Tiefenwirkung der Stimulation.

Das Verbindungselement kann, zumindest in Geradeausblickrichtung des Trägers der Stimulationsvorrichtung gesehen, vorzugsweise auch in transversale Blickrichtung gesehen, eine im wesentlichen L-förmige Kontur aufweisen. Gleichzeitig ist es vorzugsweise relativ zur Oberfläche des Kopfes zumindest soweit konvex aufgebogen, dass es eine Berührung mit der an dieser Stelle auslaufenden Helix des Ohres vermeidet.

Der Elektrodenträger kann zumindest teilweise aus elastischem Material, insbesondere aus biokompatiblem Kunststoffmaterial, besonders bevorzugt aus Silikon, aus Polyethylen, aus Polypropylen oder aus Polyurethan, bestehen. Die mindestens zwei Elektroden können dabei in das Material des Elektrodenträgers teilweise eingelagert sein. Bei diesem teilweise elastischen Material verläuft die Weg-Druck-Kurve relativ flach. Der Druck auf die Haut der Cymba steigt also bei zunehmender Vorspannung vergleichsweise langsam, da sich der Elektrodenträger durch den Andruck verbreitert und immer mehr Fläche für die Krafteinleitung zur Verfügung steht. Dies kommt einerseits dem Tragekomfort entgegen und gewährleistet andererseits eine gute Kontaktqualität für die Applizierung des Stimulationsstroms.

Der Haltebügel kann entlang seines bügelförmigen Verlaufs eine Nut zur Platzierung eines Stromkabels aufweisen. Dies ist allerdings nur eine Möglichkeit der Führung des Kabels zur Stromversorgung der Elektroden. Anstelle der Nut können auch andere Befestigungsmöglichkeiten vorgesehen werden, z. B. per Clips oder Ösen, die entlang der Führungsstrecke des Kabels (beispielsweise im Abstand jeweils eines Zentimeters) an der Stimulationsvorrichtung angeordnet sind.

Der Elektrodenträger weist, insbesondere als Elektrodenkopf ausgebildet, bevorzugt mindestens eine Stimulationselektrode und mindestens eine Referenzelektrode auf.

Die Elektroden sind bevorzugt Metallelektroden, insbesondere Titanelektroden. Sie weisen bevorzugt die Form eines Kugelabschnitts oder eines Abschnitts eines Ellipsoids auf.

In einer weiteren Ausführungsform kann der Elektrodenträger aus elektrisch leitfahigem oder elektrisch leitfähig machbarem Material bestehen. In diesem Fall ist er in zwei Abschnitte mit unterschiedlicher elektrischer Polung und einem dazwischenliegenden Isolationsabschnitt aufgeteilt.

Die Teile der Stimulationsvorrichtung sind bevorzugt - soweit Hautkontakt gegeben ist - auswechselbar und bestehen aus einem weichen Material, wobei speziell an ein Elastomermaterial gedacht ist, insbesondere an Silikon oder an ein Material, das Silikon aufweist. Im Falle des Elektrodenträgers kann die leichte Auswechselbarkeit dadurch erreicht werden, dass das Kabel am Elektrodenträger einsteckbar gestaltet ist.

Vorteilhaft ist es, dass durch die vorgeschlagene Ausgestaltung der Elektrodenanordnung eine recht stabile Konstruktion erreicht wird, ohne den Tragekomfort negativ zu beeinflussen. Die Elektroden werden stabil und zuverlässig in der benötigten Position gehalten, auch dann, wenn versehentlich eine Zugspannung auf das zuführende Kabel ausgeübt wird (zum Beispiel bei Kopfdrehung oder Armbewegungen, die das Kabel mitnehmen).

Ein Großteil der erfindungsgemäßen Otoplastik ist bei bestimmungsgemäßem Gebrauch durch die Ohrmuschel verdeckt, die Stigmatisierung des Trägers mithin relativ gering. Sichtbar ist nur ein Teil des Verbindungselements, das über die auslaufende Helix der Pinna hinwegführt.

Zudem kann die Linienführung des Designs der erfindungsgemäßen Otoplastik weich und anschmiegsam gestaltet werden, was ihr eine optisch und haptisch sympathische Anmutung verleiht. Durch ihre fast um 270° gebogene Ausführung ist eine wirkungsvolle Zugentlastung des Kabels gewährleistet.

Durch die Austauschbarkeit der aus weicherem Material hergestellten Komponenten ist eine rasche und bequeme Möglichkeit zur individuellen Anpassung durch Auswahl verschiedener Größen gegeben, ferner ein hoher Hygienestandard.

Die genannte scharnierartige Verschwenkbarkeit ist ergonomisch vorteilhaft, um den Elektrodenträger mit seinen Elektroden zuverlässig und einfach in Position bringen zu können, nachdem der Haltebügel hinter das Ohr eingehängt ist.

Ein weiterer Vorteil besteht darin, dass der Anwender seine Otoplastik durch individuelles Zurechtbiegen und Zurechtdrücken, insbesondere des Haltebügels, präzise auf Anatomie, Kontur und Relief seines eigenen Ohres und Schädels einstellen kann.

Vorteilhaft ist es weiterhin, dass durch die vorgeschlagene Ausgestaltung der Stimulationsvorrichtung eine gute Kontaktqualität der Elektroden bei der Auflage auf der Haut gegeben ist, ohne dass es eines hohen Anlagedrucks der Elektroden auf der Haut des Patienten bedarf.

In der Zeichnung ist ein Ausführungsbeispiel der Erfindung dargestellt. Es zeigen:
- Fig. 1: die Ansicht eines Ohrs mit einer Stimulationsvorrichtung, die am Ohr angeordnet ist,
- Fig. 2: die Seitenansicht der Stimulationsvorrichtung gemäß Fig. 1, teilweise geschnitten dargestellt,
- Fig. 3: die Seitenansicht des unteren Teils der Stimulationsvorrichtung gemäß Fig. 2,
- Fig. 3a: den Schnitt A-B gemäß den Fig. 3 und
- Fig. 4: den unteren Teil der Stimulationsvorrichtung in der Ansicht "C" gemäß Fig. 3.

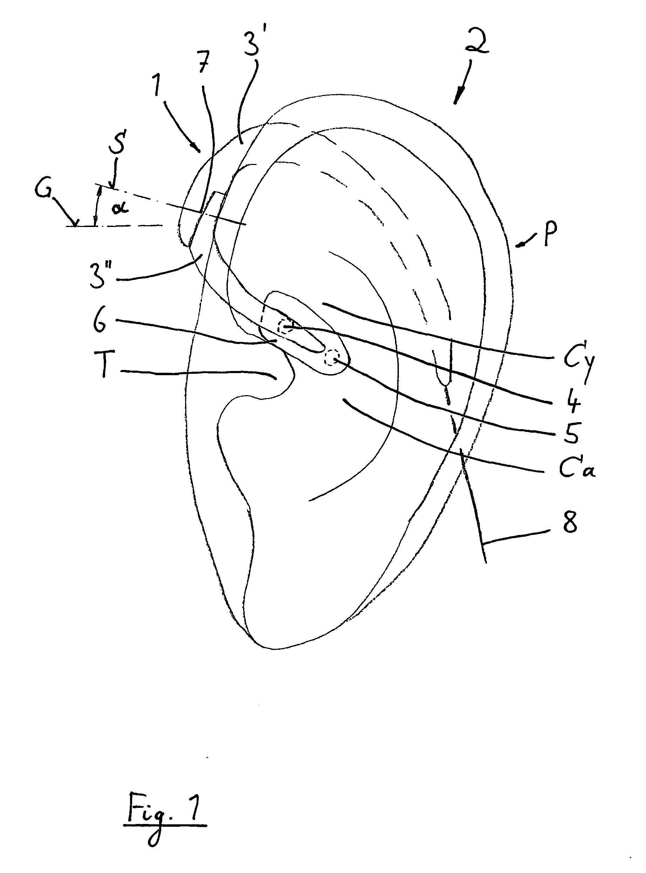

In Fig. 1 ist ein Ohr 2 eines Menschen dargestellt, in das eine Stimulationsvorrichtung 1 eingesetzt ist, um eine transkutane elektrische Stimulation vornehmen zu können. Hiermit soll bevorzugt der Vagusnerv einer Elektrostimulation unterzogen werden, um verschiedenartige Krankheiten zu behandeln bzw. Effekte zu erzielen. Mit der Stimulationsvorrichtung 1 kann demgemäß konkret auf einen Oberflächenbereich des Ohres eine transkutane elektrische Nervenstimulation vorgenommen werden. Hierfür weist die Stimulationsvorrichtung eine Stimulationselektrode und eine Referenzelektrode (s. unten) auf, zwischen denen ein elektrisches Potential erzeugt wird; die hierfür nötigen Mittel sind im Stand der Technik hinlänglich bekannt, so dass sie hier nicht weiter beschrieben werden müssen. Exemplarisch wird auf die DE 10 2005 003 735 B4 der Anmelderin verwiesen und hierauf ausdrücklich Bezug genommen.

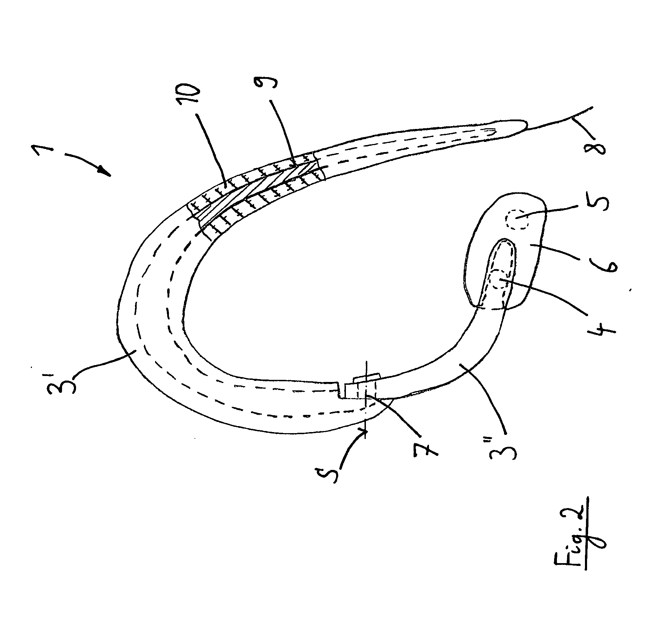

Die Stimulationsvorrichtung weist - wie es im weiteren in den Figuren 2 bis 4 zu sehen ist - ein Halteelement 3 auf, das einen Elektrodenträger 6 trägt. Am Elektrodenträger 6 sind zwei Elektroden 4 und 5 angeordnet, wobei die Elektrode 4 eine Stimulationselektrode und die Elektrode 5 eine Referenzelektrode ist. Die beiden Elektroden 4, 5 werden durch das elektrisch isolierende Material des Elektrodenträgers 6 voneinander elektrisch isoliert, um die Potentialdifferenz zwischen den Elektroden 4, 5 aufbauen zu können. Zum Aufbau der Stimulationsvorrichtung wird ausdrücklich auf die DE 10 2010 054 165 B3 der Anmelderin Bezug genommen, die hierzu Details zeigt.

In Fig. 1 ist die Pinna P des Ohrs 2 zu sehen. Der Elektrodenträger 6 kommt im Bereich der Cymba conchae Cy zu liegen; zur Orientierung eingezeichnet ist noch das Cavum conchae Ca und der Tragus T.

Wesentlich für die Stimulationsvorrichtung 1 ist folgendes: Das Halteelement 3 für den Elektrodenträger 6 besteht aus zwei Teilen, nämlich aus einem Haltebügel 3' und einem Verbindungselement 3". Der Haltebügel 3' ist sichelförmig ausgebildet, um hinter die Pinna eingehängt werden zu können (s. Fig. 1), d. h. er wird zwischen der Oberfläche des Kopfs und der Pinna P des Trägers der Stimulationsvorrichtung 1 platziert. Das Verbindungselement 3" ist zwischen dem Haltebügel 3' und dem Elektrodenträger 6 angeordnet, d. h. es verbindet diese beiden Komponenten der Stimulationsvorrichtung.

Zwischen dem Haltebügel 3' und dem Verbindungselement 3" ist ein Scharniergelenk 7 angeordnet. Bei bestimmungsgemäßer Benutzung der Stimulationsvorrichtung 1, d. h. wenn der Haltebügel 3' hinter die Pinna P eingehängt ist, wird durch das Scharniergelenk 7 eine Verschwenkung des Verbindungselements 3" relativ zum Haltebügel 3' um eine Schwenkachse S möglich, die im wesentlichen in Geradeausblickrichtung G des Trägers der Stimulationsvorrichtung 1 weist. Bei aufrecht stehendem Patienten und gerade gehaltenem Kopf würde diese Geradeausblickrichtung G also horizontal weisen und senkrecht auf dem Brustkorb des Patienten stehen. Allerdings kann zu dieser Richtung G auch noch insofern eine kleine Abweichung bestehen, als dass die Schwenkachse S zur Richtung G einen kleinen Winkel α von bis zu 30° einschließen kann.

Um die Schwenkachse S kann das Verbindungselement 3" gegen den Haltebügel 3' so in Richtung des Inneren des Kopfes verschwenkt werden (d. h. in transversale Richtung), dass, mit dem Haltebügel 3' als Widerlager, ein Andruck des Elektrodenträgers 6 gegen die Cymba Cy aufgebaut werden kann.

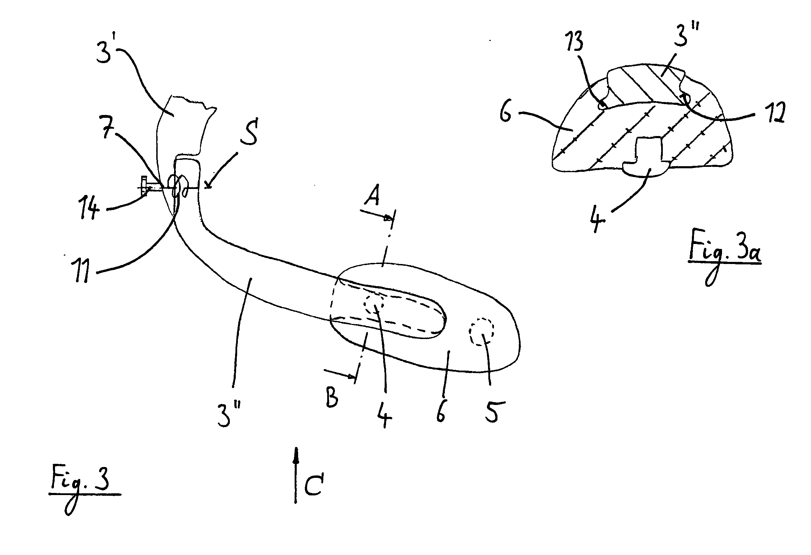

Zu diesem Zweck ist zwischen den beiden Teilen 3' und 3"im Bereich des Scharniergelenks 7 ein Federelement 11 platziert (s. Fig. 3). Das Federelement 11 stellt sicher, dass bei bestimmungsgemäßer Benutzung der Stimulationsvorrichtung 1 das Verbindungselement 3" mit endseitig befestigtem Elektrodenträger 6 mit leichtem Druck auf die Hautoberfläche im Bereich der Cymba conchae Cy drückt, so dass - ohne die Hautoberfläche des Trägers der Vorrichtung zu stark zu belasten - eine gute Kontaktqualität der Elektroden 4, 5 auf der Haut gegeben ist. Gegebenenfalls können die Elektroden auch von porösem elektrisch leitendem Material umgeben werden, um einen besonders hohen Tragekomfort zu erzielen.

Um die Vorspannung des Federelements 11 einzustellen, sind nur schematisch angedeutete Mittel 14 vorhanden. Dieses Mittel kann beispielsweise eine Rändelschraube sein, mit der das Federelement 11 - ausgebildet als Torsionsfeder - mit seiner Federwirkung wirksam zwischen den beiden Teilen 3' und 3" einstellbar ist.

Eine solche Lösung ermöglicht es beispielsweise auch, dass zunächst, vor dem Anlegen der Stimulationsvorrichtung am Ohr, das Verbindungselement 3" samt Elektrodenträger 6 vom Ohr weggeschwenkt wird, bis der Haltebügel 3' hinter die Pinna P eingehängt ist. Dann erst kann durch Drehen der Rändelschraube 14 das Federelement 11 so verspannt werden, dass der Elektrodenträger 6 mit einem als angenehm empfundenen Druck gegen die Hautoberfläche gedrückt wird.

Es ist aber auch möglich, dass die Vorspannung des Federelements per Rändelschraube 14 so eingestellt wird, wie sie der späteren Benutzung der Stimulationsvorrichtung 1 entspricht. Dann wird zum Einhängen des Haltebügels 3' hinter die Pinna P das Verbindungselement 3" gegen die Elastizität des Federelements 11 weg gehalten; nach dem Loslassen des Verbindungselements 3" liegt dann der Elektrodenträger 6 mit der als angenehm empfundenen Vorspannung auf der Hautoberfläche im Bereich der Cymba conchae Cy auf.

Der Elektrodenträger 6 ist starr, allerdings vorzugsweise auswechselbar am einen Ende des Verbindungselements 3" angeordnet. Hierzu ist eine formschlüssige Verbindung nach dem Prinzip "Nut und Feder" vorgesehen, wobei eine Schwalbenschwanznut 12 vorgesehen ist, die in den Elektrodenträger 6 eingearbeitet ist (s. Fig. 3a). In diese greift ein kongruent geformter Endabschnitt des Verbindungselements 3" mit Vorsprüngen 13 als Gegenstück ein, um den Elektrodenträger 6 starr am Verbindungselement 3" festzulegen.

In Fig. 2 ist zu sehen, dass der Haltebügel 3' aus einer Seele 9 aus Metall besteht, der von Ummantelungsmaterial 10 umgeben ist. Damit ist es möglich, den Haltebügel 3' beliebig biegbar zu gestalten, um ihn der individuell gewünschten Form anzupassen. Durch die Ummantelung - vorzugsweise aus biokompatiblem, weichem Kunststoff - ist allerdings ein hoher Tragekomfort sichergestellt.

Das Stromkabel 8 zur Versorgung der Elektroden 4, 5 mit Strom stellt die Verbindung zu einem nicht dargestellten Steuergerät her. Allerdings sei angemerkt, dass auch im Wege der Miniaturisierung das Steuergerät gegebenenfalls samt Energieversorgung auch im Haltebügel 3' untergebracht werden könnte.

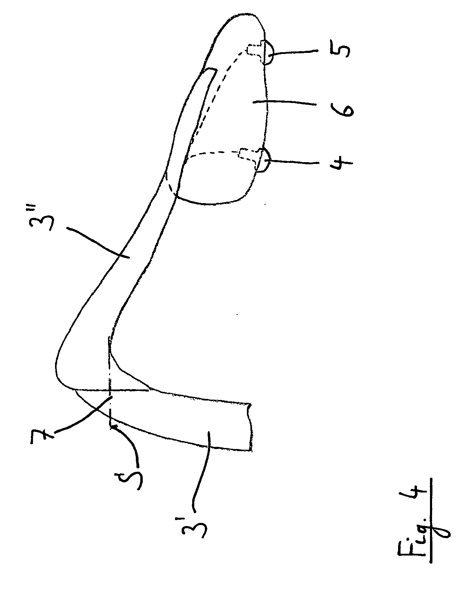

Fig. 4 als Ansicht C gemäß Fig. 3 verdeutlicht, dass das Halteelement 3 mit seinen Bestandteilen Haltebügel 3' und Verbindungselement 3" relativ zur Oberfläche des Kopfes zumindest soweit konvex aufgegebogen ist, dass es eine Berührung mit der an dieser Stelle auslaufenden Helix des Ohres (nicht dargestellt) vermeidet.

### Bezugszeichenliste:

- 1: Stimulationsvorrichtung
- 2: Ohr
- 3: Halteelement
- 3': Haltebügel
- 3": Verbindungselement
- 4: Elektrode (Stimulationselektrode)
- 5: Elektrode (Referenzelektrode)
- 6: Elektrodenträger
- 7: Scharniergelenk
- 8: Stromkabel
- 9: Seele (Metalleinsatz)
- 10: Ummantelungsmaterial
- 11: Federelement
- 12: Nut (Schwalbenschwanznut)
- 13: Gegenstück zur Nut (Vorsprung)
- 14: Mittel zur Einstellung der Vorspannung

- S: Schwenkachse
- G: Geradeausblickrichtung
- α: Winkel

- Ca: Cavum conchae
- Cy: Cymba conchae
- T: Tragus
- P: Pinna

## Patentansprüche

1. Stimulationsvorrichtung (1) zur Aufbringung eines transkutanen elektrischen Stimulationsreizes auf die Oberfläche eines Abschnittes des menschlichen Ohrs (2), die ein am oder im Ohr (2) anbringbares Halteelement (3) sowie mindestens zwei Elektroden (4, 5) aufweist, die in oder an einem Elektrodenträger (6) angeordnet sind,
wobei das Halteelement (3) für den Elektrodenträger (6) aus zwei Teilen (3', 3") besteht,
wobei das eine Teil (3') als Haltebügel ausgebildet ist, der zum Platzieren zwischen der Oberfläche des Kopfs und der Pinna (P) des Trägers der Stimulationsvorrichtung (1) ausgebildet ist,
wobei das andere Teil (3") als Verbindungselement zwischen dem Haltebügel (3') und dem Elektrodenträger (6) ausgebildet ist,
**dadurch gekennzeichnet, dass** zwischen dem Haltebügel (3') und dem Verbindungselement (3") ein Scharniergelenk (7) angeordnet ist, das bei bestimmungsgemäßer Benutzung der Stimulationsvorrichtung (1) eine Verschwenkung des Verbindungselements (3") relativ zum Haltebügel (3') um eine Achse (S) erlaubt, die parallel oder unter einem Winkel (α) von weniger als 30° zur Geradeausblickrichtung (G) des Trägers der Stimulationsvorrichtung (1) ist.

2. Stimulationsvorrichtung nach Anspruch 1, **dadurch gekennzeichnet, dass** das Scharniergelenk (7) mittels eines Federelements (11) in eine Verschwenkrichtung vorgespannt ist.

3. Stimulationsvorrichtung nach Anspruch 2, **dadurch gekennzeichnet, dass** Mittel vorhanden sind, mit denen die Vorspannung des Federelements (11) eingestellt werden kann.

4. Stimulationsvorrichtung nach einem der Ansprüche 1 bis 3, **dadurch gekennzeichnet, dass** der Elektrodenträger (6) starr am einen Ende des Verbindungselements (3") befestigt ist, wobei die Befestigung vorzugsweise als lösbare Befestigung ausgebildet ist.

5. Stimulationsvorrichtung nach Anspruch 4, **dadurch gekennzeichnet, dass** die Befestigung des Elektrodenträgers (6) am Verbindungselement (3") über eine formschlüssige Verbindung, insbesondere über eine Schwalbenschwanznut (12), erfolgt, die in den Elektrodenträger (6) oder in das Verbindungselement (3") eingearbeitet ist und die mit einem kongruenten Gegenstück (13) zusammenwirkt, die am Verbindungselement (3 ") oder am Elektrodenträger (6) angeformt ist.

6. Stimulationsvorrichtung nach einem der Ansprüche 1 bis 5, **dadurch gekennzeichnet, dass** der Haltebügel (3') sichelförmig ausgebildet ist und an seinem einen Ende das Scharniergelenk (7) angeordnet ist.

7. Stimulationsvorrichtung nach einem der Ansprüche 1 bis 6, **dadurch gekennzeichnet, dass** der Haltebügel (3') eine Seele (9) aus plastisch verformbarem Material, insbesondere aus Metall, aufweist, die von einem Ummantelungsmaterial (10), insbesondere von einem biokompatiblen Kunststoffmaterial, besonders bevorzugt von Silikon, von Polyamid, von Polypropylen oder von Polyurethan, umgeben ist.

8. Stimulationsvorrichtung nach einem der Ansprüche 1 bis 7, **dadurch gekennzeichnet, dass** das Verbindungselement (3 "), zumindest in Geradeausblickrichtung (G) des Trägers der Stimulationsvorrichtung (1) gesehen, vorzugsweise auch in transversale Blickrichtung gesehen, eine im wesentlichen L-förmige Kontur aufweist.

9. Stimulationsvorrichtung nach einem der Ansprüche 1 bis 8, **dadurch gekennzeichnet, dass** der Elektrodenträger (6) zumindest teilweise aus elastischem Material, insbesondere aus biokompatiblem Kunststoffmaterial, besonders bevorzugt aus Silikon, aus Polyethylen, aus Polypropylen oder aus Polyurethan, besteht, wobei die mindestens zwei Elektroden (4, 5) in das Material des Elektrodenträgers teilweise eingelagert sind.

10. Stimulationsvorrichtung nach einem der Ansprüche 1 bis 9, **dadurch gekennzeichnet, dass** der Haltebügel (3') entlang seines bügelförmigen Verlaufs eine Nut zur Platzierung eines Stromkabels (8) aufweist.

## Claims

1. Stimulation device (1) for the application of a transcutaneous electric stimulation stimulus onto the surface of a section of the human ear (2), which comprises a holding element (3) which can be attached at or in the ear (2) as well as at least two electrodes (4, 5) which are arranged in or at an electrode carrier (6),
wherein the holding element (3) for the electrode carrier (6) consists of two parts (3', 3 "),
wherein one of the parts (3') is designed as holding bow which is designed for the placement between the surface of the head and the Pinna (P) of the wearer of the stimulation device (1),
wherein the other part (3") is designed as connection element between the holding bow (3') and the electrode carrier (6),
**characterized in that**
between the holding bow (3') and the connection element (3") a hinge joint (7) is arranged which during intended use of the stimulation device (1) allows a swivel movement of the connection element (3") relatively to the holding bow (3') around an axis (S) which is parallel or under an angle (α) of less than 30° to the straight ahead viewing direction (G) of the wearer of the stimulation device (1).

2. Stimulation device according to claim 1, **characterized in that** the hinge joint (7) is pre-loaded by means of a spring element (11) in a swivel direction.

3. Stimulation device according to claim 2, **characterized in that** means are arranged by which the pre-load of the spring element (11) can be adjusted.

4. Stimulation device according to one of claims 1 to 3, **characterized in that** the electrode carrier (6) is rigidly fixed at one end of the connection element (3"), wherein the fixation is preferably designed as releasable fixation.

5. Stimulation device according to claim 4, **characterized in that** the fixation of the electrode carrier (6) at the connection element (3") takes place via a form-fit connection, especially by means of a dovetail groove (12), which is machined into the electrode carrier (6) or in the connection element (3") and which cooperates with a congruent counterpart (13), which is arranged at the connection element (3") or at the electrode carrier (6).

6. Stimulation device according to one of claims 1 to 5, **characterized in that** the holding bow (3') is designed sickle shaped and that the hinge joint (7) is arranged at one of its ends.

7. Stimulation device according to one of claims 1 to 6, **characterized in that** the holding bow (3') comprises a core (9) made of plastically deformable material, especially of metal, which is encased by a casing material (10), especially by a bio-compatible plastic material, specifically preferred by silicone, by polyamide, by polypropylene or by polyurethane.

8. Stimulation device according to one of claims 1 to 7, **characterized in that** the connection element (3") comprises a substantial L-shaped form, seen at least in straight ahead viewing direction (G) of the wearer of the stimulation device (1), preferably also seen in transversal viewing direction.

9. Stimulation device according to one of claims 1 to 8, **characterized in that** the electrode carrier (6) consists at least partially of elastic material, especially of bio-compatible plastic material, specifically preferred of silicone, of polyethylene, of polypropylene or of polyurethane, wherein the at least two electrodes (4, 5) are at least partially encased by the material of the electrode carrier.

10. Stimulation device according to one of claims 1 to 9, **characterized in that** the holding bow (3') comprises a groove along its bow-shaped run for the placement of an electric cable (8).

## Revendications

1. Ensemble de stimulation (1) destiné à appliquer une excitation électrique transcutanée de stimulation sur la surface d'une partie de l'oreille humaine (2), l'ensemble présentant un élément de maintien (3) apte à être placé sur ou dans l'oreille (2) ainsi qu'au moins deux électrodes (4, 5) disposées dans ou sur un porte-électrodes (6),
l'élément de maintien (3) du porte-électrodes (6) étant constitué de deux parties (3', 3''),
une partie (3') étant configurée comme étrier de maintien configuré pour être placé entre la surface de la tête et le pavillon (P) du porteur de l'oreille de l'ensemble de stimulation (1),
l'autre partie (3'') étant configurée comme élément de liaison entre l'étrier de maintien (3') et le porte-électrodes (6),
**caractérisé en ce que**
entre l'étrier de maintien (3') et l'élément de liaison (3'') est disposée une articulation de charnière (7) qui, lorsque l'ensemble de stimulation (1) est utilisé correctement, permet un pivotement de l'élément de liaison (3'') par rapport à l'étrier de maintien (3') autour d'un axe (S) qui est parallèle ou forme un angle (α) de moins de 30° par rapport à la direction d'observation rectiligne (G) du porteur de l'ensemble de stimulation (1).

2. Ensemble de stimulation selon la revendication 1, **caractérisé en ce que** l'articulation de charnière (7) est précontrainte dans une direction de pivotement au moyen d'un élément élastique (11).

3. Ensemble de stimulation selon la revendication 2, **caractérisé en ce qu'**il présente des moyens qui permettent d'ajuster la précontrainte de l'élément élastique (11).

4. Ensemble de stimulation selon l'une des revendications 1 à 3, **caractérisé en ce que** le porte-électrodes (6) est fixé de manière rigide à une extrémité de l'élément de liaison (3''), la fixation étant de préférence configurée comme fixation libérable.

5. Ensemble de stimulation selon la revendication 4, **caractérisé en ce que** la fixation du porte-électrodes (6) sur l'élément de liaison (3'') s'effectue par l'intermédiaire d'une liaison en correspondance géométrique, en particulier par l'intermédiaire d'une rainure en queue d'aronde (12) ménagée dans le porte-électrodes (6) ou dans l'élément de liaison (3'') et coopérant avec une pièce complémentaire congruente (13) formée sur l'élément de liaison (3'') ou sur le porte-électrodes (6).

6. Ensemble de stimulation selon l'une des revendications 1 à 5, **caractérisé en ce que** l'étrier de maintien (3') est configuré en forme de croissant, l'articulation de charnière (7) étant disposée à l'une de ses extrémités.

7. Ensemble de stimulation selon l'une des revendications 1 à 6, **caractérisé en ce que** l'étrier de maintien (3') présente un câble (9) en matériau déformable plastiquement, en particulier en métal, entouré par une matière de gaine (10), en particulier par une matière synthétique biocompatible et de façon particulièrement préférable en silicone, polyamide, polypropylène ou polyuréthane.

8. Ensemble de stimulation selon l'une des revendications 1 à 7, **caractérisé en ce que** l'élément de liaison (3''), au moins dans la direction d'observation rectiligne (G) du porteur de l'ensemble de stimulation (1) présente un contour essentiellement en forme de L, de préférence aussi vu dans une direction d'observation transversale.

9. Ensemble de stimulation selon l'une des revendications 1 à 8, **caractérisé en ce que** le porte-électrodes (6) est réalisé au moins en partie en matériau élastique, en particulier en une matière synthétique biocompatible, de façon particulièrement préférable en silicone, polyéthylène, polypropylène ou polyuréthane, les deux ou plusieurs électrodes (4, 5) étant incorporées en partie dans le matériau du porte-électrodes.

10. Ensemble de stimulation selon l'une des revendications 1 à 9, **caractérisé en ce que** l'étrier de maintien (3') présente le long de sa forme d'étrier une rainure permettant de placer un câble de courant (8).
